# EUROPÄISCHE PATENTANMELDUNG

(11) **EP 1 972 320 A1**
(43) Veröffentlichungstag der Anmeldung: **24.09.2008**
(21) Anmeldenummer: 07104702.1
(22) Anmeldetag: 22.03.2007
(51) Int. Cl.: A61K 6/027

(54) **Niobhaltiges Infiltrationsglas**

(71) Anmelder: Vita Zahnfabrik H. Rauter GmbH & Co. KG, 79713 Bad Säckingen (DE)
(72) Erfinder: Die Erfindernennung liegt noch nicht vor
(74) Vertreter: Meyers, Hans-Wilhelm

(57) **Zusammenfassung**

Die vorliegende Erfindung betrifft einen vollkeramischen Zahnersatz, bei dem ein poröses keramisches Gerüstmaterial mit einem Glas infiltriert wird und eine Löslichkeit von < 1100 µg/cm² nach DIN EN ISO 6872 besitzt. Gegenstand der Erfindung ist auch die Verwendung von niobhaltigem Glas mit einem Nb₂O₅-Gehalt von mehr als 0,1 Gew. % als Infiltrationsglas für vollkeramische Dentalmassen.

## Beschreibung

Die vorliegende Erfindung betrifft ein niobhaltiges Infiltrationsglas, insbesondere für keramische Körper, einen keramischen Körper einsetzbar als vollkeramischer Zahnersatz, bei dem ein poröses keramisches Gerüstmaterial mit einem Glas infiltriert wird.

Vollkeramische Massen werden heutzutage im Frontzahnbereich, als Inlays, Onlays, Veneers und Seitenzahnbrücken eingesetzt.

EP-B-0 241 384 beschreibt ein Verfahren, bei dem Metalloxidpartikel porös gesintert werden und das entstandene Gerüst anschließend mit einem Glas infiltriert wird. Das gesinterte Gerüst besteht hauptsächlich aus Aluminiumoxid und/oder Zirkonoxid. Als Infiltrationsglas wird ein alkalisches Borosilicatglas, das Aluminiumoxid enthält, beschrieben. In den Beispielen ist eine der Hauptkomponenten des Glases Lanthanoxid. Der Ausdehnungskoeffizient soll leicht unterhalb des porösen Unterbaus liegen.

Das DE-B-100 61 630 offenbart ein Verfahren, bei dem Cer-stabilisiertes Zirkonoxid und Mischungen von Cer-stabilisierten Zirkonoxid mit Aluminiumoxid als Gerüstmaterial verwendet werden. Außerdem wird ein Glas mit der Zusammensetzung 15-35 Gew.% La₂O₃, 10-25 Gew.% SiO₂,10-25 Gew.% AI₂O₃, 5-20 Gew.% B₂O₃, 5-20 Gew.% CaO, 0-10 Gew% ZrO₂, 0-10 Gew.% TiO₂ und 0-15 Gew.% CeO₂ beansprucht, sowie Zusätze an weiteren ZrO₂-stabilisierenden Metalloxiden, insbesondere 0-10 Gew.% MgO und 0-10 Gew.% Y₂O₃.

DE 692-T-06 256 beschreibt des weiteren Gerüstmaterialien auf Basis von Aluminium-/Magnesiumoxid-Spinellen.

Der Erfindung liegt die Aufgabe zugrunde, ein Glas zu entwickeln, dass sich in die bekannten vollkeramische Gerüste infiltrieren lässt und verbesserte mechanische und chemische Beständigkeiten besitzt. Die bislang eingesetzten Gläser sind durch verschiedene Randbedingungen eingeschränkt. Die Infiltrationstemperatur muss unterhalb der Sintertemperatur des Gerüstes liegen, am besten unterhalb 1200 °C. Der Ausdehnungskoeffizient muss geringfügig unter dem Wert des Gerüstes liegen. Das Glas darf keine ungewünschte Farbe aufweisen. Das Glas muss sich fehlerfrei infiltrieren lassen. Das Glas soll hohe mechanische und chemische Beständigkeiten aufweisen. Insbesondere die Limitierung der Infiltrationstemperatur und damit der Viskosität der glasbildenden Schmelze stehen der chemischen und mechanischen Stabilität entgegen, da Gläser mit einem hohen Netzwerkbildneranteil (z. B. SiO₂, B₂O₃) sehr gute mechanische und chemische Beständigkeiten aufweisen, aber erst bei höheren Temperaturen die notwendige Viskosität von ca. 10³ dPas erreichen.

Das bislang verwendete hoch-lanthan-haltige Glas lässt sich zwar hervorragend infiltrieren, zeigt bei einer exakten Anpassung im Ausdehnungsverhalten gute mechanische Eigenschaften, ist im Bereich der chemischen Stabilität im Sinne der Dentalnormen jedoch nur als Gerüstmaterial ausreichend.

Überraschenderweise zeigte sich, dass die Verwendung von Nioboxid sowohl als Zusatz zu lanthan-haltigen Gläsern als auch als Bestandteil in weiteren Silicat-Systemen eine deutliche Verbesserung in der chemischen Beständigkeit hervorruft. Das der Erfindung zu Grunde liegende Problem wird gelöst durch ein Infiltrationsglas für keramische Körper auf silicatischer Basis, wobei das Infiltrationsglas Niob in größeren als natürlich vorkommenden Mengen aufweist. In den erfindungsgemäßen Infiltrationsgläsern kann das Nb in Mengen von mindestens 0,1 Gew.%, berechnet als Nb₂O₅ und bezogen auf die Gesamtmenge des Infiltrationsglases vorkommen. Insbesondere kann beispielsweise das System SiO₂- Nb₂O₅-Na₂O/K₂O 10-70 Gew.% SiO₂, 1-40 Gew.% Na₂O/K₂O und 1-60 Gew.% Nb₂O₅ aufweisen.

Das erfindungsgemäße Infiltrationsglas zeigte bei Auslagerungen als definierter Glasgrieß (Kornfraktion 100-200 µm, in Anlehnung an die DIN ISO 719) in 4 % Essigsäure bei 80 °C für 16 h eine Verringerung der Glaslöslichkeit auf unter 3% der bekannten Gläser. Die mechanischen Eigenschaften und die weiteren Spezifikationen werden vom Nioboxid nicht negativ beeinflusst. Das erfindungsgemäße Infiltrationsglas weist insbesondere eine Löslichkeit von < 1100 µg/cm², insbesondere < 900 µg/cm² bzw.< 700 µg/cm² nach DIN EN ISO 6872 auf.

In einer Ausführungsform der Erfindung weist das die erfindungsgemäße Infiltrationsglas Zusätze von Gerüstbestandteilen auf, insbesondere Al₂O₃, CeO₂, Y₂O₃ und/oder ZrO₂.

In einer anderen Ausführungsform weist das erfindungsgemäße Infiltrationsglas aus dem System SiO₂-La₂O₃-Al₂O₃-B₂O₃ mit 15-35 Gew.% La₂O₃, 10-25 Gew.% SiO₂, 10-25 Gew.% Al₂O₃, 5-20 Gew.% B₂O₃, 5-20 Gew.% CaO, 0-10 Gew.% ZrO₂, 0-10 Gew.% TiO₂ und 0-15 Gew.% CeO₂ zusätzlich Nb₂O₅ in einer Menge von 0,1-20 Gew.% auf.

Das erfindungsgemäße Infiltrationsglas kann weitere Metalloxide in einer Oxidationsstufe, die zur Stabilisierung von ZrO₂ geeignet ist, enthalten, wobei insbesondere 0-10 Gew.% MgO und/oder 0-10 Gew.% Y₂O₃ zugemischt werden können.

Das erfindungsgemäße Infiltrationsglas kann weiterhin farbgebende Oxide enthalten.

Das erfindungsgemäße Infiltrationsglas kann typischerweise als Pulver angefertigt und vertrieben werden.

Das Pulver kann dann z.B. in an sich bekannter Weise zur Infiltration poröser Keramikkörper verwendet werden. Gegenstand der Erfindung sind mithin auch keramische Körper, die mit dem erfindungsgemäßen Infiltrationsglas infiltriert worden sind, z.B. Dentalrestaurationen.

Gegenstand der Erfindung ist weiterhin auch die Verwendung von niobhaltigem Glas mit einem Nb₂O₅-Gehalt von mehr als 0,1 Gew % als Infiltrationsglas für vollkeramische Dentalmassen. Nb₂O₅ kann insbesondere in Mengen von 0,1 bis 20 Gew.%, oder 5 bis 15 Gew.% in dem erfindungsgemäßen Infiltrationsglas vorhanden sein.

### Beispiele:

### Beispiel 1:

Ein Glas aus dem SiO₂-Nb₂O₅-Na₂O-B₂O₃-System mit Zusätzen von Aluminium-, Zirkon- und Ceroxid zur Verwendung für Gerüste aus Aluminiumoxid und Cer-stabilisierten Zirkonoxid, so dass es zu keinen nennenswerten Auflösungs- bzw. Abscheidungsprozessen vom Gerüst ins Glas, respektive aus dem Glas kommt.

Das Glas mit der Zusammensetzung 29,5 Gew.% SiO₂, 28,7 Gew.% Nb₂O₅, 12,8 Gew.% Na₂O, 6,8 Gew.% B₂O₃, 8,0 Gew.% Al₂O₃, 6,6 Gew% ZrO₂ und 7,6 Gew.% CeO₂ zeigt nach einer Auslagerung in 4 % Essigsäure nach 16 h bei 80 °C einen Gewichtsverlust von 3,5 mg pro Gramm Einwaage. Der Ausdehnungskoeffizient α₂₀₋₃₀₀ liegt bei 8,3 10⁻⁶ K⁻¹, angepasst an ein Gerüst von 50 Gew.% Cer-stabilisiertes Zirkonoxid und 50 Gew.% Aluminiumoxid. Das momentan am häufigsten eingesetzte Infiltrationsglas für dentale Vollkeramiken (In Ceram^{©}-Zirconia-Glas) hat einen Gewichtsverlust von ca. 120 mg/g_{Einwaage}.

### Beispiel 2:

Das Glas mit der Zusammensetzung 29,9 Gew.% SiO₂, 37,0 Gew.% Nb₂O₅, 11,1 Gew.% Na₂O, 7,5 Gew.% K₂O, 6,7 Gew% ZrO₂ und 7,7 Gew.% CeO₂ zeigt nach einer Auslagerung in 4 % Essigsäure nach 16 h bei 80 °C einen Gewichtsverlust von 9,2 mg pro Gramm Einwaage. Der Ausdehnungskoeffizient α₂₀₋₃₀₀ liegt bei 9,3·10⁻⁶ K⁻¹ ist in diesem Fall an ein reines Cer-stabilisiertes-Zirkonoxid-Gerüst angepasst.

### Beispiel 3:

Das Glas mit der Zusammensetzung 21,5 Gew.% SiO₂, 11,0 Gew.% B₂O₃, 17,5 Gew.% AI₂O₃, 5,5 Gew.% CaO, 31 Gew.% La₂O₃, 4 Gew.% TiO₂, 9,5 Gew.% Nb₂O₅ zeigt eine chemische Löslichkeit von 653 µg/cm² nach DIN EN ISO 6872 nach Infiltration in einen VITA^{©} In-Ceram Classic ALUMINA BLANK^{©}. Im Vergleich hierzu zeigt das bisher auf dem Markt erhältliche VITA In-Ceram^{©} ALUMINA GLASS POWDER eine chemische Löslichkeit von etwa 1200 µg/cm².

## Patentansprüche

1. Infiltrationsglas, insbesondere für keramische Körper auf silicatischer Basis, **dadurch gekennzeichnet, dass** Niob in Mengen größer als den natürlich vorkommenden Mengen enthalten ist.

2. Infiltrationsglas nach Anspruch 1, wobei Niob in Mengen größer als 0,1 Gew.%, berechnet als Nb₂O₅ und bezogen auf die Gesamtmenge des Infiltrationsglases vorkommt, insbesondere in Mengen von 1 bis 60 Gew.%.

3. Infiltrationsglas nach Anspruch 1 und/oder 2 aus dem System SiO₂-Nb₂O₅-Na₂O/K₂O mit 10-70 Gew.% SiO₂, 1-60 Gew.% Nb₂O₅,1-40 Gew.% Na₂O/K₂O.

4. Infiltrationsglas nach mindestens einem der Ansprüche 1 bis 3, das Zusätze von Bestandteile des zu infiltrierenden Gerüsts aufweist, insbesondere Al₂O₃, CeO₂, Y₂O₃ und/oder ZrO₂.

5. Infiltrationsglas nach Anspruch 1 aus dem System SiO₂-La₂O₃-Al₂O₃-B₂O₃ mit 15-35 Gew.% La₂O₃, 10-25 Gew.% SiO₂, 10-25 Gew.% Al₂O₃, 5-20 Gew.% B₂O₃, 5-20 Gew.% CaO, 0-10 Gew.% ZrO₂, 0-10 Gew.% TiO₂ und 0-15 Gew.% CeO₂ mit einer Zugabe von Nb₂O₅ von 0,1-20 Gew.%.

6. Infiltrationsglas nach mindestens einem der Ansprüche 1 bis 5, wobei das Infiltrationsglas weitere Metalloxide in einer Oxidationsstufe zur Stabilisierung von ZrO₂ enthält.

7. Infiltrationsglas nach mindestens einem der Ansprüche 1 bis 6, wobei das Infiltrationsglas 0-10 Gew.% MgO und/oder 0-10 Gew.% Y₂O₃ enthält.

8. Infiltrationsglas nach mindestens einem der Ansprüche 1 bis 7, wobei das Infiltrationsglas farbgebende Oxide enthält.

9. Infiltrationsglas nach mindestens einem der Ansprüche 1 bis 8 mit einer Löslichkeit von < 1100 µg/cm² nach DIN EN ISO 6872.

10. Keramischer Körper erhältlich durch Infiltration eines porösen keramischen Körpers mit einem Infiltrationsglas nach mindestens einem der Ansprüche 1 bis 9.

11. Keramischer Körper nach Anspruch 10, **dadurch gekennzeichnet, dass** der keramische Körper eine Dentalrestauration ist.

12. Verwendung von niobhaltigem Infiltrationsglas nach mindestens einem der Ansprüche 1 bis 9 mit einem Nb-Gehalt von mehr als 0,1 Gew % als Infiltrationsglas für keramische Körper.

13. Verwendung von niobhaltigem Infiltrationsglas nach mindestens einem der Ansprüche 1 bis 9 mit einem Nb-Gehalt von mehr als 0,1 Gew % als Infiltrationsglas für vollkeramische Dentalmassen.
